# EUROPÄISCHE PATENTSCHRIFT

(11) **EP 3 749 999 B1**
(45) Veröffentlichungstag und Bekanntmachung des Hinweises auf die Patenterteilung: **26.06.2024**
(21) Anmeldenummer: 19702612.3
(22) Anmeldetag: 01.02.2019
(51) Int. Cl.: G02B 23/24, A61B 1/00, A61B 1/04, G02B 5/04

(54) **OPTISCHES SYSTEM EINES ENDOSKOPS, ENDOSKOP, STEREO-VIDEOENDOSKOP UND VERFAHREN ZUM HERSTELLEN EINES OPTISCHEN SYSTEMS**
OPTICAL SYSTEM OF AN ENDOSCOPE, ENDOSCOPE, STEREO-VIDEO ENDOSCOPE, AND METHOD FOR PRODUCING AN OPTICAL SYSTEM
SYSTÈME OPTIQUE D'UN ENDOSCOPE, ENDOSCOPE, ENDOSCOPE VIDÉO STÉRÉOSCOPIQUE ET PROCÉDÉ DE FABRICATION D'UN SYSTÈME OPTIQUE

(30) Priorität: 06.02.2018 DE 102018102641
(43) Veröffentlichungstag der Anmeldung: 16.12.2020
(73) Patentinhaber: OLYMPUS WINTER & IBE GMBH, 22045 Hamburg (DE)
(72) Erfinder: ZHAO, Dr. Jianxin, 22399 Hamburg (DE)
(74) Vertreter: Seemann & Partner Patentanwälte mbB
(86) Internationale Anmeldenummer: PCT/EP2019/052515
(87) Internationale Veröffentlichungsnummer: WO 2019/154733

(56) Entgegenhaltungen:
- EP-A1- 3 276 390
- JP-A- 2003 149 414
- US-A- 5 689 365
- US-A1- 2004 177 913
- US-B1- 6 704 145
- US-B1- 7 280 283

## Beschreibung

Die Erfindung betrifft ein optisches System eines Endoskops mit seitlicher Blickrichtung, umfassend eine seitwärts blickende distale optische Baugruppe und eine proximale optische Baugruppe, wobei aus einem Objektraum einfallende Lichtbündel von den optischen Baugruppen entlang eines Strahlengangs geführt und auf einen Bildsensor abgebildet werden, wobei die distale optische Baugruppe eine Umlenkprismengruppe umfasst, die in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst, und wobei das zweite Prisma eine zweite Eintrittsseite, eine Reflexionsseite und eine zweite Austrittsseite umfasst. Ferner betrifft die Erfindung ein Endoskop mit seitlicher Blickrichtung sowie ein Stereo-Videoendoskop mit seitlicher Blickrichtung. Schließlich betrifft die Erfindung ein Verfahren zum Herstellen eines optischen Systems eines Endoskops mit seitlicher Blickrichtung, umfassend eine seitwärts blickende distale optische Baugruppe und eine proximale optische Baugruppe, wobei aus einem Objektraum einfallende Lichtbündel von den optischen Baugruppen entlang eines Strahlengangs geführt und auf einen Bildsensor abgebildet werden, wobei die distale optische Baugruppe eine Umlenkprismengruppe umfasst, die in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst und wobei das zweite Prisma eine zweite Eintrittsseite, eine Reflexionsseite und eine zweite Austrittsseite umfasst.

Endoskope, beispielsweise Video-Endoskope, ferner beispielsweise Stereo-Video-Endoskope, umfassen ein optisches System, mit dem an einer distalen Spitze eines Endoskopschafts eintretende Lichtbündel auf ein oder mehrere Bildsensoren abgelenkt werden. Bei Endoskopen mit seitlicher Blickrichtung unterscheidet sich die Blickrichtung des optischen Systems von einer Längsaxialrichtung des Endoskopschafts und schließt mit dieser einen vorgegebenen oder veränderlichen Winkel von typischerweise zwischen 25° und 70° ein. Es sind also sowohl Endoskope mit fester seitlicher Blickrichtung als auch Endoskope mit variabler seitlicher Blickrichtung, die auch als V-DOV-Endoskope bezeichnet werden, bekannt.

Bei Stereo-Videoendoskopen werden mit Hilfe des optischen Systems ein stereoskopisches Bildpaar und/oder zwei stereoskopische Videokanäle aufgenommen. Mit solchen Instrumenten ist es möglich, ein 3D-Abbild eines distal vor dem Ende des Endoskopschafts liegenden Objekts in einem Untersuchungs- und/oder Operationsraum zu erzeugen.

Das optische System solcher Endoskope umfasst eine Umlenkprismengruppe aus mehreren Prismen, die die aus einem Objektraum unter einem bestimmten Winkel zur Längsachse des Endoskopschafts in das optische System eintretenden Lichtstrahlen mehrfach reflektieren und seitenrichtig in Richtung eines linken und eines rechten Linsensystemkanals umlenken. Die Linsensystemkanäle umfassen jeweils einen Bildsensor zum Erfassen der Bilddaten.

Eine Umlenkprismengruppe eines Endoskops ist beispielsweise aus der EP 2 593 827 B1 bekannt.

Die Umlenkprismengruppe eines Stereo-Videoendoskops umfasst typischerweise zwei Prismen, die an ihrer gemeinsamen Grenzfläche miteinander verkittet sind. Es findet eine Reflexion der einfallenden Lichtbündel an zwei sowohl zur optischen Achse der Eintrittslinse als auch zur Längsachse des Endoskopschafts schräg stehenden und reflektierenden Grenzflächen des zweiten Prismas statt. Das zweite Prisma befindet sich in Lichteinfallsrichtung hinter dem ersten Prisma, welches unmittelbar hinter einer Eintrittslinse angeordnet ist. Die letzte Reflexion, bevor die Lichtstrahlen anschließend in eine proximale optische Baugruppe, welche aus einem oder mehreren Linsensystemkanälen bestehen kann, eintreten, findet typischerweise unter Totalreflexion statt. Mit anderen Worten findet also diese letzte Reflexion an einer Grenzfläche Glas-Luft statt.

EP 3 276 390 A1 zeigt ein optisches System und ein chirurgisches Instrument mit einem solchen optischen System, das eine im Strahlengang angeordnete Prismengruppe umfasst, welche das Sichtfeld des optischen Systems an zumindest einer Seite begrenzt.

US 2004/0177913 A1 zeigt ein Verfahren zur Herstellung einer Luftspalte zwischen zwei optischen Elementen. Dazu werden zwei Abstandshalter an den Kanten der optischen Elemente geformt und zwischen diesen Abstandshaltern ein Klebstoff aufgetragen, so dass die optischen Elemente zusammengeklebt werden können.

US 7,280,283 B1 zeigt verschiedene optische Systeme von Endoskopen. Durch den Einsatz verschiedener Linsen und optischer Elemente werden Verzerrungen und Wölbungen bei der Abbildung reduziert.

US 6,704,145 B1 zeigt eine Luftspaltenstruktur zwischen zwei optischen Elementen, die mittels Abstandselementen erzeugt wird.

JP 2003-149414 A zeigt eine Prismenanordnung, bei der die Größe eines Luftspalts zwischen zwei Prismen durch die Dicke von Abstandshaltern bestimmt wird.

In US 5,689,365 A1 ist ein Stereo-Videoendoskop mit einem optischen System gezeigt, das eine seitwärtsblickende distale optische Baugruppe umfasst.

Aufgabe der vorliegenden Erfindung ist es, ein optisches System eines Endoskops mit seitlicher Blickrichtung, ein Endoskop mit seitlicher Blickrichtung, ein Stereo-Videoendoskop mit seitlicher Blickrichtung sowie ein Verfahren zum Herstellen eines optischen Systems eines Endoskops mit seitlicher Blickrichtung anzugeben, wobei eine Umlenkprismengruppe dieses optischen Systems konstruktiv vereinfacht ist und gleichzeitig eine hohe Bildqualität erreicht.

Die Aufgabe wird gelöst durch ein optisches System eines Endoskops mit seitlicher Blickrichtung, umfassend eine seitwärts blickende distale optische Baugruppe und eine proximale optische Baugruppe, wobei aus einem Objektraum einfallende Lichtbündel von den optischen Baugruppen entlang eines Strahlengangs geführt und auf einen Bildsensor abgebildet werden, wobei die distale optische Baugruppe eine Umlenkprismengruppe umfasst, die in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst, und wobei das zweite Prisma eine zweite Eintrittsseite, eine Reflexionsseite und eine zweite Austrittsseite umfasst, wobei dieses optische System dadurch fortgebildet ist, dass die erste Austrittsseite des ersten Prismas und/oder die zweite Eintrittsseite des zweiten Prismas in einem Bereich außerhalb des Strahlengangs mit einer Beschichtung versehen ist und im Bereich des Strahlengangs zwischen der ersten Austrittsseite und der zweiten Eintrittsseite ein Luftspalt vorhanden ist, wobei die Beschichtung eine Chrom-Beschichtung oder eine zum zumindest überwiegenden Teil chromhaltige Beschichtung ist, wobei sich die mit der Beschichtung versehene Seite aus dem Bereich außerhalb des Strahlengangs und einem im Strahlengang liegenden Bereich zusammensetzt, wobei eine Grenze zwischen diesen beiden Bereichen durch diejenigen Strahlenbündel definiert ist, welche von einem Rand eines Blickfelds in das optische System einfallen und gerade noch auf den Bildsensor abgebildet werden, wobei ausschließlich der Bereich außerhalb des Strahlengangs und nicht der im Strahlengang liegende Bereich mit der Beschichtung versehen ist.

Die Konstruktion des optischen Systems gemäß Aspekten der Erfindung beruht auf den folgenden Überlegungen. An einer zweiten Eintrittsseite des zweiten Prismas der Umlenkprismengruppe findet Totalreflexion statt, bevor die Lichtbündel in die proximale optische Baugruppe, beispielsweise einen linken und einen rechten Linsensystemkanal, eingekoppelt werden. Für die erforderliche Totalreflexion ist ein Sprung im Brechungsindex zwischen dem Material des zweiten Prismas und einem sich anschließenden Medium an der entsprechenden Grenzfläche erforderlich. Für Totalreflexion gut geeignet ist eine Grenzfläche Glas-Luft.

Der erforderliche Luftspalt wird gemäß dem Stand der Technik erzeugt, indem zwischen den beiden Prismen eine Maske angeordnet wird, so dass zwischen der ersten Austrittsseite und der zweiten Eintrittsseite ein Luftspalt vorhanden ist. Gleichzeitig soll der Bauraum des optischen Systems möglichst klein sein. Daher wird die Maske mit möglichst geringer Materialstärke ausgeführt. Die Herstellung einer sehr dünnen Maske ist jedoch technisch aufwendig. Auch die Montage des optischen Systems ist eine technische Herausforderung, da die Handhabung einer derart dünnen Maske nur mit größter Vorsicht erfolgen kann und viel Erfahrung erfordert. Es ist schwierig, die Maske bei der Montage richtig zu platzieren und vor allen Dingen nicht zu beschädigen.

Die an der zweiten Eintrittsseite stattfindende Totalreflexion beansprucht lediglich einen Teil dieser Seite des Prismas. Mit anderen Worten muss der Sprung im Brechungsindex, der durch den Materialübergang Glas-Luft bereitgestellt wird, nicht vollflächig realisiert sein. Es ist ausreichend, wenn dieser Sprung in einem Bereich des Strahlengangs vorliegt. Außerhalb des Strahlengangs, also an Orten wo keine Totalreflexion stattfindet, befindet sich gemäß dem Stand der Technik die Maske.

Bereiche der ersten Austrittsseite und der zweiten Eintrittsseite innerhalb und außerhalb des Strahlengangs des optischen Systems sollen im Kontext der vorliegenden Beschreibung wie folgt definiert sein. Entlang des Strahlengangs verlaufen diejenigen Strahlenbündel, die von dem optischen System auf eine lichtempfindliche Fläche eines Bildsensors abgebildet werden.

Das optische System gemäß Aspekten der Erfindung umfasst insbesondere zumindest einen Bildsensor, wobei aus einem in einem Objektraum gelegenen Bildfeld einfallende Lichtbündel von der distalen und der proximalen optischen Baugruppe auf die lichtempfindliche Fläche des zumindest einen Bildsensors abgebildet werden.

Die im Strahlengang verlaufenden Lichtbündel durchstoßen die Grenzflächen der im optischen System vorhandenen optischen Komponenten. Derjenige Bereich einer solchen Grenzfläche, der von den Lichtbündeln durchstoßen wird, wird als im Strahlengang liegender Bereich angesehen. Diejenigen Bereiche der Oberflächen, welche nicht von den Strahlenbündeln durchstoßen werden, liegen dementsprechend außerhalb des Strahlengangs. Die Grenze zwischen diesen beiden Bereichen ist durch diejenigen Strahlenbündel definiert, welche vom Rand des Bildfelds in das optische System einfallen und gerade noch auf den zumindest einen Bildsensor abgebildet werden.

Anstatt einer Maske wird gemäß Aspekten der Erfindung eine Beschichtung vorgesehen, um den gewünschten Luftspalt zwischen der ersten Austrittsseite und der zweiten Eintrittsseite herzustellen. Wahlweise wird die Beschichtung auf die erste Austrittsseite des ersten Prismas oder auf die zweite Eintrittsseite des zweiten Prismas in einem Bereich außerhalb des Strahlengangs aufgebracht. Es ist ebenso vorgesehen, dass eine Beschichtung sowohl auf die erste Außenseite des ersten Prismas als auch auf die zweite Eintrittsseite des zweiten Prismas aufgebracht wird. Mit Hilfe dieser Beschichtung wird sichergestellt, dass zwischen der ersten Austrittsseite und der zweiten Eintrittsseite der Luftspalt vorhanden ist und an der zweiten Eintrittsseite Totalreflexion stattfindet.

Das optische System gemäß Aspekten der Erfindung ist konstruktiv vereinfacht, denn es kann auf die fragile und äußerst dünne Maske verzichtet werden. Dies vereinfacht die Herstellung und die Montage des optischen Systems. Die vorgesehene Beschichtung kann beispielsweise mit Hilfe eines geeigneten Dünnschichtverfahrens, so wie Aufdampfen oder auch Sputterdeposition, aufgebracht werden. Ein solcher Herstellungsschritt ist technisch gut und zuverlässig beherrschbar, zumal der zu beschichtende Bereich nicht mit höchster Präzision beachtet werden muss. Die gewünschte technische Funktionalität, nämlich die Herstellung des Luftspaltes, ist auch gewährleistet, wenn die Beschichtung nicht exakt an der vorgesehenen Position aufgebracht wird. Einzig entscheidend ist, dass die Beschichtung in einem Bereich außerhalb des Strahlengangs vorliegt. Es ist also insbesondere vorgesehen, dass der außerhalb des Strahlengangs liegende Bereich der ersten Austrittsseite und/oder der zweiten Eintrittsseite lediglich bereichsweise beschichtet wird.

Gemäß einer vorteilhaften Ausführungsform ist das optische System dadurch fortgebildet, dass die Beschichtung ausschließlich auf der ersten Austrittsseite des ersten Prismas oder auf der zweiten Eintrittsseite des zweiten Prismas, insbesondere ausschließlich auf der ersten Austrittsseite des ersten Prismas, vorhanden ist. Indem die Beschichtung exklusiv wahlweise auf der ersten Austrittsseite des ersten Prismas oder auf der zweiten Eintrittsseite des zweiten Prismas vorgesehen wird, ist es nicht erforderlich, zwei Oberflächen zu beschichten. Die Beschichtung auf der ersten Austrittsseite des ersten Prismas anzubringen, hat sich in der Praxis als besonders effizient herausgestellt.

Gemäß einer weiteren Ausführungsform ist vorgesehen, dass die Beschichtung eine Schichtdicke zwischen 2µm und 20µm, insbesondere zwischen 5µm und 15µm, ferner insbesondere zwischen 5µm und 10µm aufweist. Um den Effekt der Totalreflexion zu erzielen, ist ein dünner Luftspalt ausreichend. Außerdem kann durch einen dünnen Luftspalt Bauraum eingespart werden. Vorteilhaft kann durch den Einsatz einer Beschichtung ein besonders dünner Luftspalt realisiert werden. Die Größe des Luftspalts liegt in einem Bereich, der bei Verwendung einer Blende nicht erreicht werden kann. Eine Größe des Luftspalts, die in einem der genannten Intervalle liegt, hat sich als besonders praktikabel herausgestellt.

Das optische System ist dadurch fortgebildet, dass die Beschichtung eine Chrom-Beschichtung oder eine zum zumindest überwiegenden Teil chromhaltige Beschichtung ist. Eine Metallbeschichtung zeigt eine gute Haftung auf Glasoberflächen. Außerdem ist Metall duktil, so dass es wenig wahrscheinlich ist, dass die Beschichtung abplatzt. Eine Chrom-Beschichtung hat sich als besonders geeignet herausgestellt.

Der Einsatz der Beschichtung erfordert vorteilhaft nur eine geringe oder überhaupt keine Anpassung des Herstellungsprozesses des optischen Systems. So können die Prismen durch eine drückende Kraft, beispielsweise mit Hilfe eines Stempels, schrittweise direkt im Prismenhalter aneinander geklebt werden. Dieser Prozess ist ähnlich oder gleich dem herkömmlichen Masken-Klebe-Prozess. Die entsprechenden Klebeverbindungen werden im Bereich der Beschichtung vorgesehen.

Gemäß einer weiteren vorteilhaften Ausführungsform ist das optische System dadurch fortgebildet, dass die proximale optische Baugruppe einen linken Linsensystemkanal und einen rechten Linsensystemkanal umfasst, die gleichartig aufgebaut sind, und die distale optische Baugruppe dazu eingerichtet ist, aus dem Objektraum einfallende Lichtbündel in den linken Linsensystemkanal und in den rechten Linsensystemkanal einzukoppeln, und wobei die distale optische Baugruppe in Lichteinfallsrichtung aufeinanderfolgend eine Eintrittslinse, die Umlenkprismengruppe und eine Austrittslinse umfasst.

Das optische System gemäß dieser Ausführungsform ist für den Einsatz in einem Stereo-Videoendoskop geeignet. Die Verwendung in einem Stereo-Videoendoskop ist besonders vorteilhaft, da die reflektierenden Flächen der Umlenkprismengruppe bei diesem Typ Endoskop vergleichsweise groß sind. Dementsprechend muss gemäß dem Stand der Technik zur Herstellung des erforderlichen Luftspalts auch eine vergleichsweise große jedoch äußerst dünne Maske verwendet werden. Die Handhabung einer solchen Maske ist sehr anspruchsvoll und gelingt nicht in allen Fällen. Dies führt zu Verzögerungen in der Produktion und zu unnötigem Ausschuss. Diese technischen Nachteile werden bei dem optischen System gemäß Aspekten der Erfindung vorteilhaft vermieden.

Die Aufgabe wird ferner gelöst durch ein Stereo-Videoendoskop mit seitlicher Blickrichtung, welches dadurch fortgebildet ist, dass dieses ein optisches System gemäß der obigen Ausführungsform umfasst.

Ferner wird die Aufgabe gelöst durch ein Endoskop mit seitlicher Blickrichtung, welches dadurch fortgebildet ist, dass dieses ein optisches System gemäß einer oder mehrerer der oben genannten Ausführungsformen umfasst.

Das Endoskop oder das Stereo-Videoendoskop haben beispielsweise eine variable seitliche Blickrichtung.

Auf das Endoskop und das Stereo-Videoendoskop treffen gleiche oder ähnliche Vorteile zu, wie sie bereits weiter oben im Hinblick auf das optische System erwähnt wurden, so dass auf Wiederholung verzichtet werden soll.

Die Aufgabe wird ferner gelöst durch ein Verfahren zum Herstellen eines optischen Systems eines Endoskops mit seitlicher Blickrichtung, umfassend eine seitwärts blickende distale optische Baugruppe und eine proximale optische Baugruppe, wobei aus einem Objektraum einfallende Lichtbündel von den optischen Baugruppen entlang eines Strahlengangs geführt und auf einen Bildsensor abgebildet werden, wobei die distale optische Baugruppe eine Umlenkprismengruppe umfasst, die in Lichteinfallsrichtung aufeinanderfolgend ein erstes Prisma und ein zweites Prisma umfasst, wobei das erste Prisma eine erste Eintrittsseite und eine gegenüber dieser geneigte erste Austrittsseite umfasst, und wobei das zweite Prisma eine zweite Eintrittsseite, eine Reflexionsseite und eine zweite Austrittsseite umfasst, wobei das Verfahren dadurch fortgebildet ist, dass die erste Austrittsseite des ersten Prismas und/oder die zweite Eintrittsseite des zweiten Prismas in einem Bereich außerhalb des Strahlengangs mit einer Beschichtung versehen werden und die Prismen derart angeordnet werden, dass im Bereich des Strahlengangs zwischen der ersten Austrittsseite und der zweiten Eintrittsseite ein Luftspalt vorhanden ist, wobei die Beschichtung eine Chrom-Beschichtung oder eine zum zumindest überwiegenden Teil chromhaltige Beschichtung ist, wobei sich die mit der Beschichtung versehene Seite aus dem Bereich außerhalb des Strahlengangs und einem im Strahlengang liegenden Bereich zusammensetzt, wobei eine Grenze zwischen diesen beiden Bereichen durch diejenigen Strahlenbündel definiert ist, welche von einem Rand eines Blickfelds in das optische System einfallen und gerade noch auf den Bildsensor abgebildet werden, wobei ausschließlich der Bereich außerhalb des Strahlengangs und nicht der im Strahlengang liegende Bereich mit der Beschichtung versehen ist.

Das Verfahren zum Herstellen des optischen Systems ist einfacher und durchführbar. Die Handhabung einer fragilen und leicht zu beschädigenden äußerst dünnen Maske entfällt. Dadurch ist die Herstellung des optischen Systems schneller, wobei zuverlässiger bessere Ergebnisse erzielt werden.

Im Übrigen treffen auf das Verfahren zum Herstellen des optischen Systems gleiche oder ähnliche Vorteile zu, wie sie bereits weiter oben im Hinblick auf das optische System selbst erwähnt wurden.

Das Verfahren ist insbesondere dadurch fortgebildet, dass die Beschichtung ausschließlich auf der ersten Austrittsseite des ersten Prismas oder auf der zweiten Eintrittsseite des zweiten Prismas, insbesondere ausschließlich auf der ersten Austrittsseite des ersten Prismas, aufgebracht wird.

Ferner ist das Verfahren insbesondere dadurch fortgebildet, dass die Beschichtung mit einer Schichtdicke zwischen 2µm und 20µm, insbesondere zwischen 5µm und 15µm, ferner insbesondere zwischen 5µm und 10µm aufgebracht wird.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte perspektivische Ansicht eines Endoskops mit seitlicher Blickrichtung,
- Fig. 2: eine schematisch vereinfachte Darstellung eines optischen Systems eines Stereo-Endoskops,
- Fig. 3: eine weitere schematisch vereinfachte Schnittansicht eines optischen Systems eines Endoskops mit seitlicher Blickrichtung,
- Fig. 4: eine schematisch vereinfachte Draufsicht auf eine zweite Eintrittsseite eines zweiten Prismas einer Umlenkprismengruppe eines optischen Systems, wobei die zweite Eintrittsseite in einem Bereich außerhalb des Strahlengangs mit einer Beschichtung versehen ist.

In den Zeichnungen sind jeweils gleiche oder gleichartige Elemente und/oder Teile mit denselben Bezugsziffern versehen, so dass von einer erneuten Vorstellung jeweils abgesehen wird.

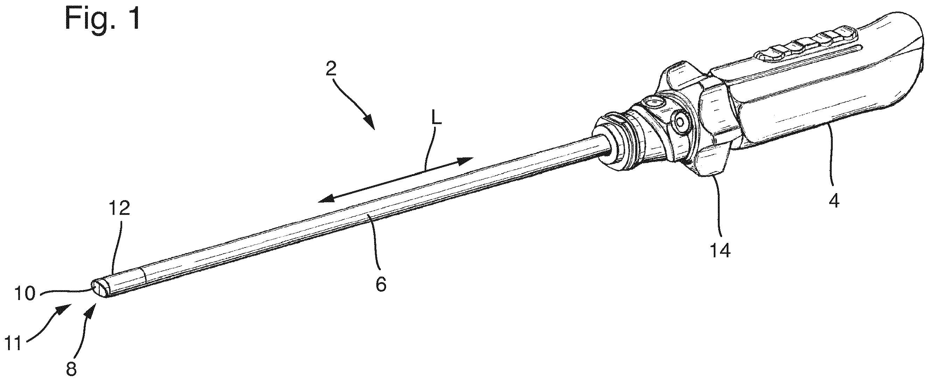

Fig. 1 zeigt in schematisch vereinfachter perspektivischer Darstellung ein Endoskop 2, umfassend einen proximalen Handgriff 4, an den sich lediglich beispielhaft ein starrer Endoskopschaft 6 anschließt. Der Endoskopschaft 6 kann ebenso flexibel oder halbflexibel ausgebildet sein. An seiner distalen Spitze 8 des Endoskopschafts 6 befindet sich ein Eintrittsfenster 10, durch welches Licht aus einem Objektraum 11, beispielsweise einem Operations- oder Beobachtungsfeld, in ein in Fig. 1 nicht sichtbares optisches System des Endoskops 2 einfällt. Das optische System ist beispielsweise in einem distalen Abschnitt 12 des Endoskopschafts 6 angeordnet. Das optische System bildet Objekte, die sich im Objektraum 11 befinden, auf zumindest einen Bildsensor ab. Zu diesem Zweck umfasst das optische System zumindest einen Bildsensor. Der oder die Bildsensor(en) ist/sind bevorzugt (ein) solche(r) mit hoher Auflösung, z.B. HD, 4K oder folgende Technologien.

Das Endoskop 2 ist beispielsweise ein chirurgisches Instrument. Es handelt sich um ein Endoskop 2 mit fester seitlicher Blickrichtung oder mit variabler seitlicher Blickrichtung. Beispielhaft ist ein Endoskop 2 mit fester seitlicher Blickrichtung dargestellt, dessen Eintrittsfenster 10 geneigt im Endoskopschaft 6 montiert ist, so dass eine optische Achse einer Eintrittslinse des in Fig. 1 nicht dargestellten optischen Systems mit einer Längserstreckungsrichtung L des Endoskopschafts 6 einen festen Winkel einschließt, der beispielsweise zwischen 25° und 70° beträgt.

Eine Änderung der Blickrichtung um die Längsachse des Endoskopschafts 6 wird beispielsweise durch eine Drehung des Handgriffs 4 bewirkt. Das im distalen Abschnitt 12 vorhandene optische System rotiert bei einer solchen Drehung des Handgriffs 4 mit. Zur Beibehaltung der Horizontallage des angezeigten Bilds wird bei einer Drehung des Handgriffs 4 ein Drehrad 14 festgehalten, was bewirkt, dass die Bildsensoren im Inneren des Endoskopschafts 6 die Drehbewegung nicht mitvollziehen.

Bei dem dargestellten Endoskop 2 handelt es sich insbesondere um ein Videoendoskop, ferner insbesondere um ein Stereo-Videoendoskop.

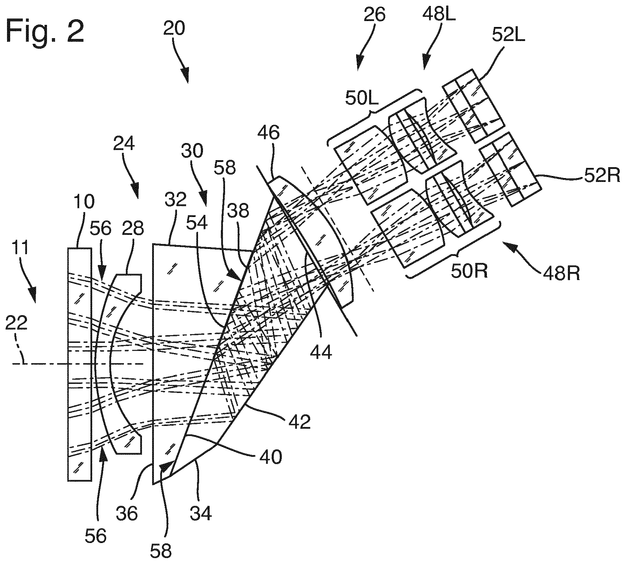

Fig. 2 zeigt ein optisches System 20, wie es in einem Stereo-Endoskop, insbesondere einem Stereo-Videoendoskop zum Einsatz kommt. Das optische System 20 definiert eine feste seitliche Blickrichtung. Die optische Achse 22, bei der es sich um die optische Achse einer Eintrittslinse 28 des optischen Systems 20 handelt, schließt mit der Längserstreckungsrichtung L des Endoskopschafts 6 einen festen Winkel von beispielsweise 30° ein. Das optische System 20 umfasst eine seitwärts blickende distale optische Baugruppe 24 und eine proximale optische Baugruppe 26. Da es sich bei dem dargestellten optischen System 20 um das System eines Stereo-Videoendoskops handelt, umfasst die proximale optische Baugruppe 26 einen linken Linsensystemkanal 48L und einen rechten Linsensystemkanal 48R. Die beiden Linsensystemkanäle 48L, 48R sind gleichartig aufgebaut. Die distale optische Baugruppe 24 ist dazu eingerichtet, aus dem Objektraum 11 einfallende Lichtbündel sowohl in den linken Linsensystemkanal 48L als auch in den rechten Linsensystemkanal 48R der proximalen optischen Baugruppe 26 einzukoppeln.

Die aus dem Objektraum 11 durch das Eintrittsfenster 10 einfallenden Lichtbündel (dargestellt in stichpunktierter Linie) treffen zunächst auf die Eintrittslinse 28 der distalen optischen Baugruppe 24 und gelangen anschließend in eine Umlenkprismengruppe 30. Die Umlenkprismengruppe 30 umfasst, betrachtet in Lichteinfallsrichtung, ein erstes Prisma 32 und ein darauffolgendes zweites Prisma 34.

In Lichteinfallsrichtung durchqueren die Lichtbündel, welche die Eintrittslinse 28 verlassen, zunächst eine erste Eintrittsseite 36 des ersten Prismas 32. Die Lichtbündel durchqueren den Körper des ersten Prismas 32 und gelangen zu dessen erster Austrittsseite 38. Die erste Austrittsseite 38 ist gegenüber der ersten Eintrittsseite 36 geneigt. Angrenzend an die erste Austrittsseite 38 befindet sich ein in Fig. 2 aufgrund seiner geringen Dimension nicht dargestellter Luftspalt 54. Die Lichtbündel durchqueren den Luftspalt 54 und treten über eine zweite Eintrittsseite 40 in das zweite Prisma 34 ein. Das zweite Prisma 34 umfasst eine Reflexionsseite 42, die gegenüber der zweiten Eintrittsseite 40 geneigt ist. Die Lichtbündel werden an der Reflexionsseite 42 des zweiten Prismas 34 reflektiert. Von dort aus gelangen sie von der Rückseite her zu der zweiten Eintrittsseite 40 des zweiten Prismas 34.

An der zweiten Eintrittsseite 44 liegt eine Grenzfläche zwischen dem Material des zweiten Prismas 34 und dem Luftspalt 54 vor, so dass an der zweiten Eintrittsseite 4 ein Sprung des Brechungsindex vorhanden ist. Die Lichtbündel werden daher von innen kommend an der zweiten Eintrittsseite 40 des zweiten Prismas 34 totalreflektiert und verlassen anschließend das zweite Prisma 34 an seiner zweiten Austrittsseite 44. Von dort aus gelangen die Lichtbündel weiter in Lichteinfallsrichtung zu einer Austrittslinse 46 der distalen optischen Baugruppe 24.

Die proximale optische Baugruppe 26 umfasst den linken Linsensystemkanal 48L und den rechten Linsensystemkanal 48R. Die beiden Linsensystemkanäle 48L, 48R sind gleichartig oder identisch aufgebaut und ferner so zueinander ausgerichtet, dass ihre jeweiligen optischen Achsen parallel zueinander liegen. Der linke Linsensystemkanal 48L umfasst die abbildende linke Linsengruppe 50L, die das einfallende Licht auf den linken Bildsensor 52L abbildet. Der rechte Linsensystemkanal 48R umfasst die abbildende rechte Linsengruppe 50R, die die einfallenden Lichtbündel auf den rechten Bildsensor 52R abbildet.

Die durch das optische System 20 verlaufenden Lichtbündel definieren innerhalb des optischen Systems 20 einen Strahlengang. Die Grenzen des Strahlengangs sind durch die Grenzlichtbündel 56 festgelegt. Die Grenzlichtbündel 56 sind diejenigen Lichtbündel, welche aus dem Objektraum 11 einfallen und gerade noch auf eine lichtempfindliche Fläche der Bildsensoren 52L, 52R abgebildet werden. Die Lichtbündel durchstoßen die einzelnen Grenzflächen der optischen Komponenten des optischen Systems 20. Dabei werden diejenigen Bereiche, welche von den Lichtbündeln durchstoßen werden, als Bereiche innerhalb des Strahlengangs angesehen. Die Bereiche, welche außerhalb liegen, also nicht mehr von Lichtbündeln durchstoßen werden, werden als außerhalb des Strahlengangs liegend angesehen. Die Grenze zwischen diesen beiden Bereichen definieren die Grenzlichtbündel 56.

Zur Herstellung des Luftspalts 54 zwischen der ersten Austrittsseite 38 und der zweiten Eintrittsseite 40 ist die erste Austrittsseite 38 des ersten Prismas 32 und/oder die zweite Eintrittsseite 40 des zweiten Prismas 34 in einem Bereich außerhalb des Strahlengangs, welcher in Fig. 2 beispielhaft mit Bezugszeichen 58 bezeichnet ist, mit einer Beschichtung versehen. Die Beschichtung hat eine Schichtdicke zwischen 2µm und 20µm und stellt sicher, dass zwischen der ersten Austrittsseite 38 und der zweiten Eintrittsseite 40 ein Luftspalt 54 vorhanden ist. Es ist ebenso vorgesehen, dass die Beschichtung ausschließlich auf der ersten Austrittsseite 38 des ersten Prismas 32 oder ausschließlich auf der zweiten Eintrittsseite 40 des zweiten Prismas 34 vorhanden ist. Bei der Beschichtung handelt es sich bevorzugt um eine Metallbeschichtung, beispielsweise um eine Chrombeschichtung oder eine Beschichtung, die zum überwiegenden Teil chromhaltig ist.

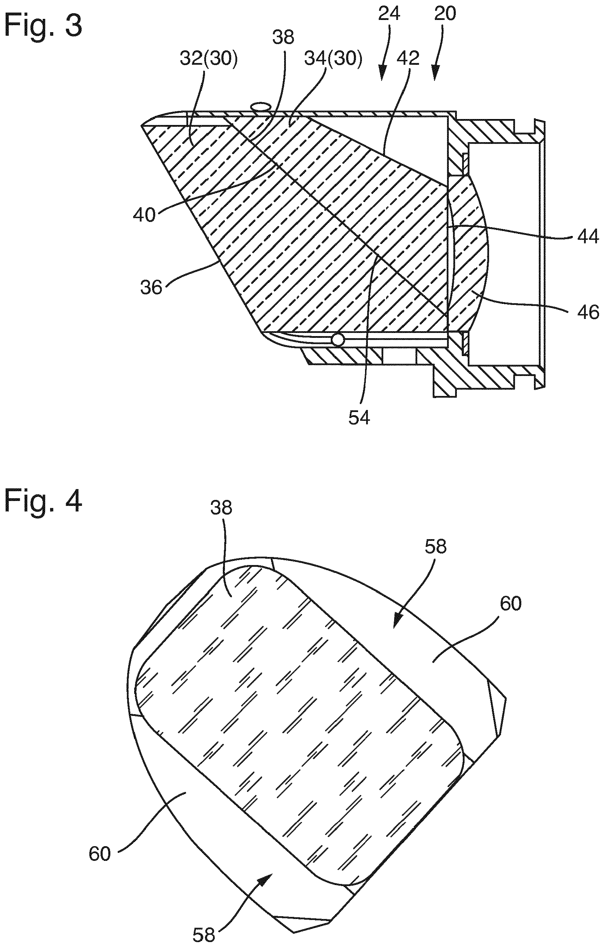

Fig. 3 zeigt einen Teil eines weiteren optischen Systems 20 eines Endoskops 2. Dargestellt ist die distale optische Baugruppe 24, wobei eine ggf. vorhandene Eintrittslinse nicht dargestellt ist. Das gezeigte optische System kommt für Bild- oder Videoendoskope zum Einsatz, welche keine Stereoabbildungen liefern. Das optische System umfasst eine Umlenkprismengruppe 30 mit einem ersten Prisma 32 und einem zweiten Prisma 34.

Aus einem Objektraum einfallende Lichtbündel durchqueren die Umlenkprismengruppe 30. Sie treten durch die erste Eintrittsseite 36 des ersten Prismas 32 in den Körper des ersten Prismas 32 ein und verlassen diesen an der ersten Austrittsseite 38. Die Lichtstrahlen durchqueren einen Luftspalt 54 zwischen der ersten Austrittsseite 38 und der zweiten Eintrittsseite 40 des zweiten Prismas 34. In dem zweiten Prisma 34 werden die Lichtbündel an der Reflexionsseite 42 des zweiten Prismas 34 reflektiert und gelangen von der Rückseite her zur zweiten Eintrittsseite 40. Dort werden sie total reflektiert, da zwischen der zweiten Eintrittsseite 40 und der ersten Austrittsseite 38 der Luftspalt 54 vorhanden ist. Die Lichtbündel verlassen die Umlenkprismengruppe 30 über die zweite Austrittsseite 44 des zweiten Prismas 34. Sie gelangen über die Austrittslinse 46 in eine in Fig. 3 nicht dargestellte proximale optische Baugruppe des optischen Systems 20, welche die Lichtbündel schließlich auf einen Bildsensor lenkt.

Fig. 4 zeigt eine Draufsicht auf die erste Austrittsseite 38 des ersten Prismas 32. In einem Bereich 58, der außerhalb des Strahlengangs liegt, ist die erste Austrittsseite 38 mit der Beschichtung 60 versehen. Die in Fig. 4 dargestellte Draufsicht gilt analog auch für das erste Prisma 32 des in Fig. 2 dargestellten optischen Systems.

Die Beschichtung 60 wird beispielsweise mit einem Dünnschichtverfahren auf die erste Austrittsseite 38 des ersten Prismas 32 aufgebracht.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: Endoskop
- 4: Handgriff
- 6: Endoskopschaft
- 8: distale Spitze
- 10: Eintrittsfenster
- 11: Objektraum
- 12: distaler Abschnitt
- 14: Drehrad
- 20: optisches System
- 22: optische Achse
- 24: distale optische Baugruppe
- 26: proximale optische Baugruppe
- 28: Eintrittslinse
- 30: Umlenkprismengruppe
- 32: erstes Prisma
- 34: zweites Prisma
- 36: erste Eintrittsseite
- 38: erste Austrittsseite
- 40: zweite Eintrittsseite
- 42: Reflexionsseite
- 44: zweite Austrittsseite
- 46: Austrittslinse
- 48L: linker Linsensystemkanal
- 48R: rechter Linsensystemkanal
- 50L: linke Linsengruppe
- 50R: rechte Linsengruppe
- 52L: linker Bildsensor
- 52R: rechter Bildsensor
- 54: Luftspalt
- 56: Grenzlichtbündel
- 58: Bereich
- 60: Beschichtung

- L: Längserstreckungsrichtung

## Patentansprüche

1. Optisches System (20) eines Endoskops (2) mit seitlicher Blickrichtung, umfassend eine seitwärts blickende distale optische Baugruppe (24) und eine proximale optische Baugruppe (26), wobei aus einem Objektraum (11) einfallende Lichtbündel von den optischen Baugruppen (24, 26) entlang eines Strahlengangs geführt und auf einen Bildsensor (52L, 52R) abgebildet werden, wobei die distale optische Baugruppe (24) eine Umlenkprismengruppe (30) umfasst, die in Lichteinfallsrichtung aufeinanderfolgend ein erstes Prisma (32) und ein zweites Prisma (34) umfasst, wobei das erste Prisma (32) eine erste Eintrittsseite (36) und eine gegenüber dieser geneigte erste Austrittsseite (38) umfasst, und wobei das zweite Prisma (34) eine zweite Eintrittsseite (40), eine Reflexionsseite (42) und eine zweite Austrittsseite (44) umfasst, **dadurch gekennzeichnet, dass** die erste Austrittsseite (38) des ersten Prismas (32) und/oder die zweite Eintrittsseite (40) des zweiten Prismas (34) in einem Bereich (58) außerhalb des Strahlengangs mit einer Beschichtung (60) versehen ist und im Bereich des Strahlengangs zwischen der ersten Austrittsseite (38) und der zweiten Eintrittsseite (40) ein Luftspalt (54) vorhanden ist, wobei die Beschichtung (60) eine Chrom-Beschichtung oder eine zum zumindest überwiegenden Teil chromhaltige Beschichtung ist, wobei sich die mit der Beschichtung (60) versehene Seite aus dem Bereich (58) außerhalb des Strahlengangs und einem im Strahlengang liegenden Bereich zusammensetzt, wobei eine Grenze zwischen diesen beiden Bereichen durch diejenigen Strahlenbündel definiert ist, welche von einem Rand eines Blickfelds in das optische System (20) einfallen und gerade noch auf den Bildsensor (52L, 52R) abgebildet werden, wobei ausschließlich der Bereich (58) außerhalb des Strahlengangs und nicht der im Strahlengang liegende Bereich mit der Beschichtung (60) versehen ist.

2. Optisches System (20) nach Anspruch 1, **dadurch gekennzeichnet, dass** die Beschichtung ausschließlich auf der ersten Austrittsseite (38) des ersten Prismas (32) oder auf der zweiten Eintrittsseite (40) des zweiten Prismas (34), insbesondere ausschließlich auf der ersten Austrittsseite (38) des ersten Prismas (32), vorhanden ist.

3. Optisches System (20) nach Anspruch 1 oder 2, **dadurch gekennzeichnet, dass** die Beschichtung (60) eine Schichtdicke zwischen 2µm und 20µm, insbesondere zwischen 5µm und 15µm, ferner insbesondere zwischen 5µm und 10µm aufweist.

4. Optisches System (20) nach einem der Ansprüche 1 bis 3, **dadurch gekennzeichnet, dass** die proximale optische Baugruppe (26) einen linken Linsensystemkanal (48L) und einen rechten Linsensystemkanal (48R) umfasst, die gleichartig aufgebaut sind, und die distale optische Baugruppe (24) dazu eingerichtet ist, aus dem Objektraum (11) einfallende Lichtbündel in den linken Linsensystemkanal (48L) und in den rechten Linsensystemkanal (48R) einzukoppeln, und wobei die distale optische Baugruppe (24) in Lichteinfallsrichtung aufeinanderfolgend eine Eintrittslinse (28), die Umlenkprismengruppe (30) und eine Austrittslinse (46) umfasst.

5. Endoskop (2) mit seitlicher Blickrichtung, **gekennzeichnet durch** ein optisches System (20) nach einem der Ansprüche 1 bis 3.

6. Stereo-Videoendoskop mit seitlicher Blickrichtung, **gekennzeichnet durch** ein optisches System (20) nach Anspruch 4.

7. Verfahren zum Herstellen eines optischen Systems (20) eines Endoskops (2) mit seitlicher Blickrichtung, umfassend eine seitwärts blickende distale optische Baugruppe (24) und eine proximale optische Baugruppe (26), wobei aus einem Objektraum (11) einfallende Lichtbündel von den optischen Baugruppen (24, 26) entlang eines Strahlengangs geführt und auf einen Bildsensor (52L, 52R) abgebildet werden, wobei die distale optische Baugruppe (24) eine Umlenkprismengruppe (30) umfasst, die in Lichteinfallsrichtung aufeinanderfolgend ein erstes Prisma (32) und ein zweites Prisma (34) umfasst, wobei das erste Prisma (32) eine erste Eintrittsseite (36) und eine gegenüber dieser geneigte erste Austrittsseite (38) umfasst, und wobei das zweite Prisma (34) eine zweite Eintrittsseite (40), eine Reflexionsseite (42) und eine zweite Austrittsseite (44) umfasst, **dadurch gekennzeichnet, dass** die erste Austrittsseite (38) des ersten Prismas (32) und/oder die zweite Eintrittsseite (40) des zweiten Prismas (34) in einem Bereich (58) außerhalb des Strahlengangs mit einer Beschichtung (60) versehen werden und die Prismen (32, 34) derart angeordnet werden, dass im Bereich des Strahlengangs zwischen der ersten Austrittsseite (38) und der zweiten Eintrittsseite (40) ein Luftspalt vorhanden ist, wobei die Beschichtung (60) eine Chrom-Beschichtung oder eine zum zumindest überwiegenden Teil chromhaltige Beschichtung ist, wobei sich die mit der Beschichtung (60) versehene Seite aus dem Bereich (58) außerhalb des Strahlengangs und einem im Strahlengang liegenden Bereich zusammensetzt, wobei eine Grenze zwischen diesen beiden Bereichen durch diejenigen Strahlenbündel definiert ist, welche von einem Rand eines Blickfelds in das optische System (20) einfallen und gerade noch auf den Bildsensor (52L, 52R) abgebildet werden, wobei ausschließlich der Bereich (58) außerhalb des Strahlengangs und nicht der im Strahlengang liegende Bereich mit der Beschichtung (60) versehen ist.

8. Verfahren nach Anspruch 7, **dadurch gekennzeichnet, dass** die Beschichtung (60) ausschließlich auf der ersten Austrittsseite (38) des ersten Prismas (32) oder auf der zweiten Eintrittsseite (40) des zweiten Prismas (34), insbesondere ausschließlich auf der ersten Austrittsseite (38) des ersten Prismas (32), aufgebracht wird.

9. Verfahren nach Anspruch 7 oder 8, **dadurch gekennzeichnet, dass** die Beschichtung (60) mit einer Schichtdicke zwischen 2µm und 20µm, insbesondere zwischen 5µm und 15µm, ferner insbesondere zwischen 5µm und 10µm aufgebracht wird.

## Claims

1. An optical system (20) of an endoscope (2) having a lateral viewing direction, comprising a sideways looking distal optical assembly (24) and a proximal optical assembly (26), wherein light bundles incident from an object space (11) are guided along a beam path by the optical assemblies (24, 26) and imaged on an image sensor (52L, 52R), wherein the distal optical assembly (24) comprises a deflection prism assembly (30) which comprises a first prism (32) and a second prism (34) which follow one another in the direction of incident light, wherein the first prism (32) comprises a first inlet side (36) and a first outlet side (38) inclined with respect thereto, and wherein the second prism (34) comprises a second inlet side (40), a reflection side (42) and a second outlet side (44), **characterized in that** the first outlet side (38) of the first prism (32) and/or the second inlet side (40) of the second prism (34) is/are provided with a coating (60) in a region (58) outside of the beam path, and an air gap (54) is provided in the region of the beam path between the first outlet side (38) and the second inlet side (40), wherein the coating (60) is a chromium coating or a coating containing, at least for the most part, chromium, wherein the side provided with the coating (60) being composed of the region (58) outside the beam path and a region inside the beam path, wherein a border between these two regions being defined by those bundles of rays which enter the optical system (20) from an edge of a field of view and are still imaged on the image sensor (52L, 52R), wherein only the region (58) outside the beam path and not the region inside the beam path is provided with the coating (60).

2. The optical system (20) according to Claim 1, **characterized in that** the coating is provided exclusively on the first outlet side (38) of the first prism (32) or on the second inlet side (40) of the second prism (34), particularly exclusively on the first outlet side (38) of the first prism (32).

3. The optical system (20) according to Claim 1 or 2, **characterized in that** the coating (60) has a layer thickness between 2µm and 20µm, particularly between 5µm and 15µm, more particularly between 5µm and 10µm.

4. The optical system (20) according to any one of Claims 1 to 3, **characterized in that** the proximal optical assembly (26) comprises a left lens system channel (48L) and a right lens system channel (48R) which have a similar design, and the distal optical assembly (24) is designed to couple light bundles incident from the object space (11) into the left lens system channel (48L) and into the right lens system channel (48R), and wherein the distal optical assembly (24) comprises an inlet lens (28), the deflection prism assembly (30) and an outlet lens (46) which follow one another in the direction of incident light.

5. An endoscope (2) having a lateral viewing direction, **characterized by** an optical system (20) according to any one of Claims 1 to 3.

6. A stereo-video endoscope having a lateral viewing direction, **characterized by** an optical system (20) according to Claim 4.

7. A method for producing an optical system (20) of an endoscope (2) having a lateral viewing direction, comprising a sideways looking distal optical assembly (24) and a proximal optical assembly (26), wherein light bundles incident from an object space (11) are guided along a beam path by the optical assemblies (24, 26) and imaged on an image sensor (52L, 52R), wherein the distal optical assembly (24) comprises a deflection prism assembly (30) which comprises a first prism (32) and a second prism (34) which follow one another in the direction of incident light, wherein the first prism (32) comprises a first inlet side (36) and a first outlet side (38) inclined with respect thereto, and wherein the second prism (34) comprises a second inlet side (40), a reflection side (42) and a second outlet side (44), **characterized in that** the first outlet side (38) of the first prism (32) and/or the second inlet side (40) of the second prism (34) is/are provided with a coating (60) in a region (58) outside of the beam path, and the prisms (32, 34) are arranged in such a manner that an air gap is provided in the region of the beam path between the first outlet side (38) and the second inlet side (40), wherein the coating (60) is a chromium coating or a coating containing, at least for the most part, chromium, wherein the side provided with the coating (60) being composed of the region (58) outside the beam path and a region inside the beam path, wherein a border between these two regions being defined by those bundles of rays which enter the optical system (20) from an edge of a field of view and are still imaged on the image sensor (52L, 52R), wherein only the region (58) outside the beam path and not the region inside the beam path is provided with the coating (60).

8. The method according to Claim 7, **characterized in that** the coating (60) is applied exclusively to the first outlet side (38) of the first prism (32) or to the second inlet side (40) of the second prism (34), particularly exclusively to the first outlet side (38) of the first prism (32).

9. The method according to Claim 7 or 8, **characterized in that** the coating (60) is applied with a layer thickness between 2µm and 20µm, particularly between 5µm and 15µm, more particularly between 5µm and 10µm.

## Revendications

1. Système optique (20) d'un endoscope (2) avec direction de vision latérale, comprenant un ensemble optique distal (24) regardant latéralement et un ensemble optique proximal (26), des faisceaux lumineux incidents proviennent d'un espace objet (11) étant guidés par les ensembles optiques (24, 26) le long d'un trajet de faisceau et étant imagés sur un capteur d'image (52L, 52R), l'ensemble optique distal (24) comprenant un groupe de prismes de déviation (30), qui comprend, successivement dans la direction d'incidence de la lumière, un premier prisme (32) et un deuxième prisme (34), le premier prisme (32) comprenant un premier côté d'entrée (36) et un premier côté de sortie (38) incliné par rapport à celui-ci, et le deuxième prisme (34) comprenant un deuxième côté d'entrée (40), un côté de réflexion (42) et un deuxième côté de sortie (44), **caractérisé en ce que** le premier côté de sortie (38) du premier prisme (32) et/ou le deuxième côté d'entrée (40) du deuxième prisme (34) sont pourvus d'un revêtement (60) dans une zone (58) située à l'extérieur du trajet des faisceaux et **en ce qu'**il existe un interstice d'air (54) dans la zone du trajet des faisceaux entre le premier côté de sortie (38) et le deuxième côté d'entrée (40), le revêtement (60) étant un revêtement de chrome ou un revêtement contenant au moins une partie prépondérante de chrome, le côté pourvu du revêtement (60) étant composé de la zone (58) située à l'extérieur du trajet des faisceaux et d'une zone située dans le trajet des faisceaux, une limite entre ces deux zones étant définie par les faisceaux de rayons qui pénètrent dans le système optique (20) à partir d'un bord d'un champ de vision et qui sont simplement imagés sur le capteur d'image (52L, 52R), seule la zone (58) située à l'extérieur du trajet des faisceaux et non la zone située dans le trajet des faisceaux étant pourvue du revêtement (60).

2. Système optique (20) selon la revendication 1, **caractérisé en ce que** le revêtement est présent exclusivement sur le premier côté de sortie (38) du premier prisme (32) ou sur le deuxième côté d'entrée (40) du deuxième prisme (34), notamment exclusivement sur le premier côté de sortie (38) du premier prisme (32).

3. Système optique (20) selon la revendication 1 ou la revendication 2, **caractérisé en ce que** le revêtement (60) présente une épaisseur de couche comprise entre 2µm et 20µm, notamment entre 5µm et 15µm, en outre notamment entre 5µm et 10µm.

4. Système optique (20) selon l'une des revendications 1 à 3, **caractérisé en ce que** l'ensemble optique proximal (26) comprend un canal (48L) de système de lentilles gauche et un canal (48R) de système de lentilles droit qui sont de même structure, et l'ensemble optique distal (24) est conçu pour relier des faisceaux lumineux incidents provenant de l'espace objet (11) dans le canal gauche (48L) de système de lentilles et dans le canal droit (48R) de système de lentilles, et l'ensemble optique distal (24) comprend, successivement dans la direction d'incidence de la lumière, une lentille d'entrée (28), le groupe de prismes de déviation (30) et une lentille de sortie (46).

5. Endoscope (2) à vision latérale, **caractérisé par** un système optique (20) selon l'une des revendications 1 à 3.

6. Vidéo-endoscope stéréoscopique à vision latérale, **caractérisé par** un système optique (20) selon la revendication 4.

7. Procédé de fabrication d'un système optique (20) d'un endoscope (2) avec direction de vision latérale, comprenant un sous-ensemble optique distal (24) regardant latéralement et un sous-ensemble optique proximal (26), des faisceaux lumineux incidents proviennent d'un espace objet (11) étant guidés par les sous-ensembles optiques (24, 26) le long d'un trajet de faisceau et étant imagés sur un capteur d'image (52L, 52R), l'ensemble optique distal (24) comprenant un groupe de prismes de déviation (30) qui comprend un premier prisme (32) et un deuxième prisme (34) se succédant dans la direction d'incidence de la lumière, le premier prisme (32) comprenant un premier côté d'entrée (36) et un premier côté de sortie (38) incliné par rapport à celui-ci, et le deuxième prisme (34) comprenant un deuxième côté d'entrée (40), un côté de réflexion (42) et un deuxième côté de sortie (44), **caractérisé, en ce que** le premier côté de sortie (38) du premier prisme (32) et/ou le deuxième côté d'entrée (40) du deuxième prisme (34) sont pourvus d'un revêtement (60) dans une zone (58) située à l'extérieur du trajet des faisceaux et les prismes (32, 34) sont disposés de telle sorte qu'il existe un interstice d'air entre le premier côté de sortie (38) et le deuxième côté d'entrée (40) dans la zone du trajet des faisceaux, le revêtement (60) étant un revêtement de chrome ou un revêtement contenant au moins une partie prépondérante de chrome, le côté pourvu du revêtement (60) se compose de la zone (58) située à l'extérieur du trajet des faisceaux et d'une zone située dans le trajet des faisceaux, une limite entre ces deux zones étant définie par les faisceaux de rayons qui pénètrent dans le système optique (20) à partir d'un bord d'un champ de vision et qui sont simplement imagés sur le capteur d'image (52L, 52R), seule la zone (58) située à l'extérieur du trajet des faisceaux et non la zone située dans le trajet des faisceaux étant pourvue du revêtement (60).

8. Procédé selon la revendication 7, **caractérisé en ce que** le revêtement (60) est appliqué exclusivement sur le premier côté de sortie (38) du premier prisme (32) ou sur le deuxième côté d'entrée (40) du deuxième prisme (34), notamment exclusivement sur le premier côté de sortie (38) du premier prisme (32).

9. Procédé selon la revendication 7 ou la revendication 8, **caractérisé en ce que** le revêtement (60) est appliqué avec une épaisseur de couche comprise entre 2µm et 20µm, notamment entre 5µm et 15µm, en outre notamment entre 5µm et 10µm.
